# EUROPEAN PATENT SPECIFICATION

(11) **EP 1 800 663 B1**
(45) Date of publication and mention of the grant of the patent: **20.01.2016**
(21) Application number: 05793605.6
(22) Date of filing: 11.10.2005
(51) Int. Cl.: A61K 9/00, A61K 47/36, A61K 47/42

(54) **ENTERAL NUTRIENT**
ENTERALER NÄHRSTOFF
NUTRIMENT ENTÉRALE

(30) Priority: 12.10.2004 JP 2004298139
(43) Date of publication of application: 27.06.2007
(73) Proprietor: Meiji Co., Ltd., Koto-ku Tokyo 136-8908 (JP)
(72) Inventor: TOYAMA, Yoshio, Odawara-shi Kanagawa 250-0862 (JP); MURAO, Kanehisa, Odawara-shi Kanagawa 250-0862 (JP)
(74) Representative: Vossius & Partner Patentanwälte Rechtsanwälte mbB
(86) International application number: PCT/JP2005/018989
(87) International publication number: WO 2006/041173

(56) References cited:
- EP-A1- 0 480 305
- EP-A1- 1 029 456
- WO-A1-00/13529
- WO-A2-00/60953
- JP-A- 61 139 361
- JP-A- 61 139 361
- JP-A- 62 224 260
- JP-A- 2000 169 397
- JP-A- 2000 169 397
- JP-A- 2003 201 230
- US-A- 4 670 268
- US-A- 5 700 513
- US-A1- 2004 197 381
- US-B1- 6 241 996
- JOANNE K. TOBACMAN: "Review of Harmful Gastrointestinal Effects of Carrageenan in Animal Experiments", ENVIRONMENTAL HEALTH PERSPECTIVES, [Online] vol. 109, no. 10, October 2001 (2001-10), pages 983-994, Retrieved from the Internet: URL:http://www.ncbi.nlm.nih.gov/pmc/articl es/PMC1242073/pdf/ehp0109-000983.pdf> [retrieved on 2014-05-19]

## Description

### Technical Field

The present invention relates to semi-solidification of an enteral nutrient, and more specifically, the present invention relates to a technique used for the purpose of solidifying or thickening an enteral nutrient or an enriched liquid food to be used by being administered to a patient through a tube in tube feeding (tube feeding infusion) such as gastrostomic feeding, and particularly relates to the use of a sodium carrageenan as a semi-solidifying agent in a technique of preventing gastroesophageal reflux in which an enteral nutrient or an enriched liquid food administered to a patient flows backward from the stomach to the esophagus of the patient.

### Background Art

For a patient unable to take food by the mouth, tube feeding (also referred to as tube feeding infusion), which is a method in which a nutrient is infused into the stomach or intestine of the patient through a feeding tube, is carried out. The nutrient to be administered to the patient by the tube feeding is defined as an enteral nutrient. This tube feeding is a method in which the enteral nutrient is administered to the patient through a tube and it includes transnasal (or nasal) tube feeding, transgastric (or intestinal) feeding and enteral (or intestinal) feeding.

With regard to the enteral nutrient, as a conventional technique to administer the enteral nutrient to a patient through the nose or the skin, it has been disclosed that a liquid food is administered as an enteral nutrient (JP-A-2000-152975).

However, when the liquid food is infused into the stomach as an enteral nutrient by, for example, the gastrostomic feeding, the liquid food flows back from the stomach and esophagus, whereby vomiting, reflux esophagitis or aspiration pneumonia is often caused. Therefore, as a method of preventing gastroesophageal reflux in the gastrostomic feeding, tube feeding infusion in which an enteral nutrient is solidified using agar is carried out (Rinsyou Kango, 29 (5) : 664-670, 2003). To be more specific, agar is dissolved in hot water at a temperature of 80°C or above and an enteral nutrient which is heated to the body temperature is added thereto and mixed to obtain homogeneity. Then, the resulting mixture is aspirated with syringes, which is solidified by cooling it in a refrigerator, and brought back to room temperature before administration and then, administration is carried out by pushing it out of the syringe.

JP-A-2000-169397 relates to a specific gelatinizer for tube-feeding nutritive food which contains a carrageenan and alginic acid.

EP-A1-1 029 456 discloses a specific antiemetic food product comprising a solution containing one or more thickeners selected from low-methoxyl pectin, sodium alginate, alginic acid, kappa carrageenan, iota carrageenan, lambda carrageenan and gellan gum and to an antiemetic food product comprising, as one set, a solution containing any of these thickeners, and typically a calcium solution.

US-B 1-6 241 996 relates to a stabilized, highly nutritional feeding composition in which substantially all of the protein component is derived from soy and is incorporated in said composition at high levels.

WO-A2-00/60953 discloses a nutritional product comprising a protein source, a lipid source and a carbohydrate source including high amylase starch and guar gum. Further disclosed is a nutritional product comprising a hydrolyzed whey protein source, a source of lipids and a sufficient amount of stabilizer to reduce serum separation, the stabilizer either including high amylase starch and guar gum or including carrageenan, starch and guar gum.

### Disclosure of the invention

The idea that an.enteral nutrient is solidified by adding agar thereto as described above is innovative and excellent. However, it is necessary to carry out a cooling treatment after a heating treatment is carried out, therefore, a burden is imposed on a care-giver for the preparation, and also it takes time, accordingly, further improvement has been demanded.

The present invention has been made in order to respond to the above-mentioned demand in this field, and has its obj ect to develop a new solidified enteral nutrient (or solidified enriched liquid food) that lightens a care-giver's burden and can be simply and easily administered within a short period of time.

Accordingly, the present inventors made studies from various viewpoints, arid as a result, they focused on the importance of a solidifying agent to be used for solidification. Then, screening for a solidifying agent (which is used for preparing a semi-solidified enteral nutrient (whose definition will be described below), therefore, it is also referred to as a semi-solidifying agent or a starch adhesive in some cases) with which a desired semi-solidified enteral nutrient can be prepared from various gelatinizing agents, thickening agents and starch adhesives was carried out intensively.

The present inventors made studies with regard to the solidification of an enteral nutrient or.an enriched liquid food using various gelatinizing agents and thickening agents, and as a result, they found out that such a nutrient or an enriched liquid food can be quickly thickened or solidified at a temperature of 45 to 40°C or below by using a starch adhesive, which contains as the main component sodium iota-carrageenan, sodium kappa-carrageenan, sodium lambda-carrageenan or a mixture thereof, without dissolving the starch adhesive by heating. Further, it was confirmed that it is an administration method almost free from water liberation which may be a cause of a leak from a fistula when the solidified product was administered from a syringe, thus the present invention was completed.

That is, a basic technical idea of the present invention is a semi-solidified enteral nutrient, which is an enteral nutrient to be infused into the body in tube feeding, characterized by being formed into a semi-solid matter using a sodium carrageenan in which the total amount of potassium, calcium and magnesium ions is 5.0% by weight or less, as a semi-solidifying agent such that the enteral nutrient has fluidity before and after the infusion thereof into the body and is not liquefied at the body temperature in a state of being retained in the body.

Thus, according to the present invention, for example, a semi-solidified enteral nutrient as described below can be provided:
A semi-solidified enteral nutrient, which is an enteral nutrient to be administered to the inside of the stomach or the intestine of a patient from a container connected outside the body of the patient to a feeding tube to be inserted into a fistula formed between the abdominal wall and the stomach wall or the intestinal wall of the patient through the feeding tube by the pressure from the container. By adding a sodium carrageenan to a liquid nutrient as a semi-solidifying agent, the semi-solidified enteral nutrient is formed as a semi-solid matter which is common with a solid matter in terms of a point that it has shape retention in which the shape does not change and is maintained in natural conditions, in terms of a property to try to maintain the.initial shape of itself against the gravity and the atmospheric pressure that naturally acts on the enteral nutrient from the outside, and in terms of a point that it can incorporate a nutritional component at a higher concentration than a liquid or a fluid, and which is common with a liquid or a fluid in terms of a point that it changes its shape by a forcible external force more easily than a solid matter. The shape retention is continuously maintained before and after the administration of the enteral nutrient to the body of the patient, and also the shape retention is maintained in such a manner that the enteral nutrient is not liquefied at the body temperature of the patient in a state that the enteral nutrient is retained in the inside of the stomach or the intestine of the patient.

The present invention further provides a method of preparing a semi-solidified enteral nutrient according to the invention, which is an enteral nutrient to be infused into the body in tube feeding infusion, comprising the step of employing a sodium carrageenan, in which the total amount of potassium, calcium and magnesium ions is 5.0% by weight or less, as a semi-solidifying agent to form a semi-solid such that the enteral nutrient has fluidity before and after the infusion thereof into the body and is not liquefied at the body temperature in a state of being retained in the body.

The present invention still further provides the use of a sodium carrageenan, in which the total amount of potassium, calcium and magnesium ions is 5.0% by weight or less, as a semi-solidifying agent for a semi-solidified enteral nutrient according to the invention, which is an enteral nutrient to be infused into the body in tube feeding infusion, which is formed into a semi-solid matter using the sodium carrageenan as a semi-solidifying agent such that the enteral nutrient has fluidity before and after the infusion thereof into the body and is not liquefied at the body temperature in a state of being retained in the body.

Incidentally, the "semi-solidified enteral nutrient" in the present invention refers to an enteral nutrient that exhibits a semi-solidified state in such a manner that the enteral nutrient has fluidity before and after the infusion thereof into the body and is not liquefied at the body temperature in a state of being retained in the body, and the details and specific embodiments are as described below.

According to the present invention, by using a sodium carrageenan in which the total amount of potassium, calcium and magnesium ions is 5.0% by weight or less, as a semi-solidifying agent (a starch adhesive), the semi-solidification of an enteral nutrient or an enriched liquid food without resort to an additional special treatment such as a heat-dissolution treatment like agar which is a conventionally used starch adhesive was successful for the first time, and the operation can be considerably simplified compared with the case of using the conventional agar. Further, it is also possible to adjust the physical property of an enteral nutrient or an enriched liquid food to be administered by the added amount of the starch adhesive, and there is also a significant effect that enteral nutrients or enriched liquid foods having different physical properties can be easily administered to the respective patients. Further, the present invention can be widely used in various tube feeding methods, and moreover, it can also be used in an administration method utilizing percutaneous endoscopic gastrostomy (PEG).

Further, by incorporating a nutritional component such as a protein, a carbohydrate and a lipid to the semi-solidifying agent, when it is added to an enteral nutrient, it becomes possible to enhance a necessary nutritional element as well as to semi-solidify it. Further, by using a semi-solidifying agent containing a necessary nutritional component as an enteral nutrient, a semi-solid enteral nutrient canbe obtained only by adding water thereto, and moreover, it is possible to adjust the physical property of the semi-solid state by the temperature or the amount of water to be added.

### Best Mode for Carrying Out the Invention

In the present invention, a sodium carrageenan in which the total amount of potassium, calcium and magnesium ions is 5.0% or less, is used as a semi-solidifying agent (a starch adhesive). It is disclosed herein that any substance can be used as long as it is soluble in cold water (at 45°C or below, preferably 40°C or below) and can semi-solidify an enteral nutrient (an enriched liquid food).

The carrageenan is a polysaccharide extracted from a seaweed belonging to the families of Hypneaceae, Solieriaceae and Gigartinaceae in the Gigartinales in red algae. The carrageenan has a property that normally it does not dissolve without heating and it gelates when a heated solution thereof is cooled, and there is not so much of a difference from agar or the like. However, as a result of further examining the carrageenan, the present inventors noticed that by controlling the total amount of potassium, calcium and magnesium ions in the carrageenan to be 5.0% by weight or less, the hydration thereof with cold water is improved. For this purpose, the carrageenan is produced as a sodium carrageenan. The carrageenan has a low content of potassium, calcium and magnesium ions by reducing them through, for example, ion exchange with an ion exchange resin. More specifically, the total amount of potassium, calcium and magnesium ions is 5.0% by weight or less. In this way, by preparing the major part of the carrageenan as any of sodium kappa-carrageenan, sodium iota-carrageenan and sodium lambda-carrageenan or a mixture thereof, the carrageenan can be hydrated in cold water within a short period of time.

In particular, the kappa-carrageenan or the iota-carrageenan originally has a solidification ability. Therefore, after it is dissolved in cold water as a sodium carrageenan, by further adding a cation or a milk protein thereto, an arbitrary solidification degree can be obtained.

Accordingly, the semi-solidifying agent (starch adhesive) to be used in the present invention is basically composed of a sodium carrageenan, and more specifically, for example, it is a mixture containing sodium lambda-carrageenan as the main component and mixed with sodium iota-carrageenan and sodium kappa-carrageenan, and the mixture is soluble in cold water (at 0 to 45°C, preferably at 10 to 40°C, more preferably at 15 to 35°C, and generally at room temperature) and reacts with a cation or a milk protein thereby to form a gel.

An enteral nutrient (an enriched liquid food) is a liquid with fluidity passing through a narrow tube with an inner diameter of 1 to 2 mm and containing a nutritional element such as a protein (milk or soybean), a lipid, a carbohydrate and a mineral at a high concentration so as to provide a high calorie with a small amount, i.e., from 1 kcal to 2 kcal per 1 ml. A commercially available product can also be appropriately used.

The source of potassium, calcium and protein to be used in the enteral nutrient varies depending on the product in many cases, and it is considered that the amount thereof to be added, the degree of ionization, and the state of dispersion in the liquid also vary. For example, the calcium source to be used in Ensure (an enteral nutrient manufactured by Abbott: trade name) is tribasic calcium phosphate, and is insoluble and is not ionized in a liquid food. On the other .hand, it is considered that those containing calcium derived from a milk protein as the main component are ionized.

In the present invention, basically, such an enteral nutrient (and/or an enriched liquid food) is treated with a sodium (hereinafter also referred to as a Na-type) carrageenan as a semi-solidifying agent to give a semi-solidified enteral nutrient, and the semi-solidifying agent may be used by directly adding it to an enteral nutrient, or may be used by dissolving the semi-solidifying agent in water and mixing it with an enteral nutrient.

As described above, in the present invention, basically, a sodium carrageenan in which the total amount of potassium, calcium and magnesium ions is 5.0% or less, is used as the semi-solidifying agent, however, in order to prepare a starch adhesive (a semi-solidifying agent) suitable for a widely used enteral nutrient (enriched liquid food), it is preferred that lambda-carrageenan, kappa-carrageenan and iota-carrageenan coexist at a suitable ratio therein, respectively. In general, each of the iota-carrageenan, lambda-carrageenan and kappa-carrageenan is not present as a single substance, and three types or two types of them coexist therein. Therefore, in order to make the ratio appropriate, it is preferred to adjust the mixing ratio of the two types of Na-type lambda-carrageenan (lambda-carrageenan accounting for about 50% and kappa-carrageenan accounting for about 50%) and Na-type iota-carrageenan (iota-carrageenan accounting for almost 100%).

Among the above two types of carrageenans, the Na-type lambda-carrageenan has high solubility in cold water, and in the case of using the sodium carrageenan by directly adding it to a liquid food as a powdered solidifying agent, it is necessary to incorporate the Na-type lambda-carrageenan therein at 30% or more. In order to further increase the solubility, the content thereof is preferably 50% or more. On the other hand, in the method in which after the powdered solidifying agent is dissolved in water, an enteral nutrient is added thereto, by incorporating the Na-type iota-carrageenan as the main component, it is possible to apply it to a wide variety of enteral nutrients. Both of the iota-carrageenan and the kappa-carrageenan have reactivity with a potassium ion and a calcium ion, however, it is known that the former reacts with a calcium ion more strongly and the latter reacts with a potassium ion more strongly. Accordingly, a gel forming ability was examined using various enteral nutrients (enriched liquid foods) manufactured by various manufacturers. As a result, it is preferred to set the ratio of the Na-type iota-carrageenan to the Na-type lambda-carrageenan to be 10 : 0 to 0 : 10, preferably 10 : 0 to 5 : 5, more preferably 10 : 0 to 7 : 3.

Incidentally, in the starch adhesive of the present invention, other than the above Na-type carrageenan, pregelatinized starch or dextrin which is soluble in cold water can be added for the purpose of strengthening the physical property thereof or improving the dispersibility thereof. Further, by adding locust bean gum or tara gum which forms a gel by reacting with the carrageenan, it is also possible to add a completely different gel forming ability. However, it is preferred that any substance has a property of solubility in cold water. Further, xanthan gum or guar gum can be used in combination. With regard to these gelatinizing agents, one or more types can be used along with the Na-type carrageenan.

In addition to the use of the starch adhesive listed above in the form of a powder, in order to increase the dispersibility or solubility in water or an enteral nutrient (an enriched liquid food), it is preferred that the starch adhesive is formed into a granule or a liquid. The starch adhesive (semi-solidifying agent) to be used in the present invention maybe used by adding an amount of from 0.1 to 15 g, preferably from 0.5 to 10 g, more preferably from 1 to 6 g as a powdered starch adhesive relative to 200 ml of the amount of the liquid to be administered (that is, an enteral nutrient + water), however, without sticking to the above range of addition, the range of addition can be appropriately determined depending on the type of the enteral nutrient or the desired gelatinizing degree of the semi-solid matter. A starch adhesive other than the Na-type carrageenan may also be used by adding an amount in the above range of addition or with reference to the range.

Other than the utilization as a starch adhesive as described above, by incorporating a nutritional component such as a protein in addition to a sodium carrageenan as a semi-solidifying agent, it becomes possible to strengthen a necessary nutritional element as well as to semi-solidify an enteral nutrient. Further, by incorporating a necessary nutritional element as an enteral nutrient, a semi-solid enteral nutrient can be obtained only by adding water thereto, and moreover, it is possible to adjust the physical property of the semi-solid state by the temperature or the amount of water to be added.

The nutritional element to be contained in the semi-solidifying agent is preferably at least one member selected from proteins, lipids, carbohydrates, dietary fibers, vitamins and minerals. More preferably, it surely contains a protein, and the protein source preferably contains casein.

Hereinafter, the present invention will be described in more detail with reference to Examples, however, the present invention is not limited only to these Examples.

### <Example 1> In the case where a Na-type carrageenan was added to Ensure

This Example was carried out in.a system in which water and Ensure (trade name) were mixed at a ratio of 1 : 1. First, 3 g of a starch adhesive (a Na-type carrageenan) was added to 100 ml of water and dissolved completely. 100 ml of Ensure was added thereto and after the mixture was stirred to homogeneity with a spatula, it was aspirated with a 100-ml syringe and the syringe was maintained at room temperature for 10 minutes. Thereafter, the solidified Ensure was pushed out of the syringe and, as the following Table 1, its property was measured with the passage of time.

"Hardness" and water liberation status were selected as evaluation items, and as for the "hardness", the semi-solid matter pushed out of the syringe was collected in a container with a diameter of 40 mm and with a height of 15 mm and the compressive stress thereof was measured by uniaxial compression measurement with RHEONER manufactured by Yamaden Co., Ltd., with a plunger with a diameter of 20 mm under conditions where the speed was 1 mm/s and the clearance was 5 mm. Further, the water liberation status was confirmed by visual observation and the amount of an aqueous liquid dropping from a strainer. The results were as shown in the following Table 1.

Even under various use conditions in this way, the physical property of the product of the present invention was stable. In particular, the physical property of the product subjected to a treatment of cooling followed by bringing it back to the room temperature is stable, therefore, the product is very useful when considering how to use the product in the medical or nursing-care field. As is clear from the following Table 3, the fact that the change in the physical property between the product right after completion of cooling (at 60 minutes after initiation of cooling) and the product brought back to normal temperature after cooling (after cooling → room temperature) was small was a unique effect of the product of the present invention which could not be arrived at from the prior art.

**(Table 1)**

| | Right after preparation | At 30 minutes after leaving at room temperature | At 60 minutes after initiation of cooling | Cooling → room temperature |
|---|---|---|---|---|
| Hardness (N/m²) | 807.9 | 614.5 | 673.8 | 683.2 |
| Water liberation | No | No | No | No |

### <Example 2> In the case where starch was added to a Na-type carrageenan

Example 2 was carried out in the same manner as in Example 1 except for the content and added amount of starch adhesive. The starch adhesive was a mixture obtained by mixing a pregelatinized starch with a Na-type carrageenan at a ratio of 1 : 1 and 7 g thereof was added. The results were as shown in Table 2.

**(Table 2)**

| | Right after preparation | At 30 minutes after leaving at room temperature | At 60 minutes after initiation of cooling | Cooling → room temperature |
|---|---|---|---|---|
| Hardness (N/m²) | 948.3 | 1086 | 1167 | 577.1 |
| Water liberation | No | No | No | No |

### <Comparative Example 1> Example of preparation with agar

Comparative Example 1 was carried out in a system in which water and Ensure were mixed at a ratio of 1 : 1 in the same manner as in the Example. However, agar was used as a semi-solidifying agent (starch adhesive).

First, 1 g of agar was added to 100 ml of water and dissolved completely. 100 ml of Ensure, which was heated to about 40°C, was added thereto and after the mixture was stirred to homogeneity with a spatula, it was aspirated with a 100-ml syringe and the syringe was maintained in a refrigerator for 30 minutes and completely solidified.

Each of the product right after completion of cooling and the product brought back to normal temperature after cooling was pushed out of the syringe and its property was measured. The results are shown in the following Table 3.

**(Table 3)**

| | At 60 minutes after initiation of cooling | Cooling → room temperature |
|---|---|---|
| Hardness (N/m²) | 555.3 | 499.1 |
| Water liberation* | 30% | 45% |

| | | |
|---|---|---|
| * Water liberation (%) = weight of liberalized water / total weight x 100 | | |

As is clear from the results shown in Examples 1 and 2 and Comparative Example 1, in Examples 1 and 2, a sufficient hardness could be achieved right after the preparation in both cases. Further, even in the case where the product was left at normal temperature for a while or cooled in a refrigerator, water liberation was not observed at all and the condition was good.

On the other hand, in Comparative Example in which agar (Kanten cook: Ina Food Industry Co., Ltd.) was used, although a certain degree of hardness could be achieved with a small amount, the water liberation was significant, and in particular, it was more significant when the product was left at normal temperature after being refrigerated.

Further, a test was carried out in the same method as described above for the following 3 types of solidifying agents (gelatinizing agents, starch adhesive) in addition to the carrageenan.
(I) xanthan gum + cold water-soluble locust bean gum
(Ro) cold water-soluble carrageenan + cold water-soluble locust bean gum
(Ha) liquid pectin

The results were as follows.
(A) In the case where a powdered gelatinizing agent is directly added to a liquid food:
   In the cases of (I) and (Ro), the solubility is extremely low and because it takes 60 minutes or more for preparation, they cannot be used.
   In the case of (Ha), it does not dissolve at all when it is in a form of a powder.
(B) In the case where a powdered gelatinizing agent is added to a liquid food after it is dissolved in water;
   (I) and (Ro) gelatinize at the time of being dissolved in water, therefore, it is difficult to be mixed to homogeneity with a liquid food. In addition, even if they are mixed to homogeneity, a sufficient hardness cannot be obtained.

Contrary to this, because the Na-type carrageenan used here gelatinizes by reacting with a protein, a potassium ion and a calcium ion in the liquid food even if the powder thereof is directly added to the liquid food or it is used after being dissolved in water, it does not have any problem as described above and it was confirmed that the Na-type carrageenan is the best gelatinizing agent.

### <Example 3> In the case where a protein, a lipid and a carbohydrate were added to a Na-type carrageenan

Examples of adjusting a physical property in the case where a protein, a lipid and a carbohydrate are incorporated in a Na-type carrageenan as a semi-solidifying agent are shown.

The raw material compositions (the composition for the verification of the physical property depending on the temperature, the composition for the verification of the physical property depending on the amount of water added) used in this Example are shown in Table 4 and Table 5, respectively.

**(Table 4)**

| Composition for verification of physical property depending on temperature | |
|---|---|
| Raw material weight [g] | Composition |
| Calcium caseinate | 2.2 |
| Skim milk | 5.9 |
| Powdered oil | 3.5 |
| Na-type lambda-carrageenan | 0.4 |
| Dextrin | 15.1 |
| Water for dissolution | 72.9 |
| Total weight [g] | 100.0 |
| Calorie [kcal] | 100 |

**(Table 5)**

| Composition for verification of physical property depending on amount of water added | |
|---|---|
| Raw material weight [g] | Composition |
| Calcium caseinate | 2.2 |
| Skim milk | 5.9 |
| Powdered oil | 6.0 |
| Na-type lambda-carrageenan | 0.4 |
| Dextrin | 38.9 |
| Total weight [g] | 53.4 |
| Calorie [kcal] | 200 |

(1) After the raw materials were dissolved in water for dissolution at 25°C, 50°C or 80°C, the physical property after the mixture was refrigerated to 12 ± 2°C was verified. The results were as shown in Table 6.

**(Table 6)**

| Change in physical property depending on temperature for dissolution | | | |
|---|---|---|---|
| Temperature of water for dissolution | 25°C | 50°C | 80°C |
| Hardness [x10³ N/m²] | 1.5 | 2.8 | 3.8 |
| Adhesiveness [x10² J/m³] | 1.1 | 3.1 | 3.3 |

As is clear from the above results, it could be verified that as the temperature of water for dissolution become higher, the hardness increases.
(2) After 53 g of a semi-solidifying agent containing a nutritional element was dissolved in 2, 3, and 4 times volume of water for dissolution at 25°C, the mixture was refrigerated to 12 ± 2°C, and then the physical property thereof was verified. The results were as shown in Table 7.

**(Table 7)**

| Change in physical property depending on amount of water added | | | |
|---|---|---|---|
| Amount of water for dissolution added [g] | 106.8 | 160.2 | 213.6 |
| Hardness [×10³ N/m²] | 1.8 | 0.6 | 0.2 |
| Adhesiveness [×10² J/m³] | 2.8 | 0.6 | 0.4 |

As is clear from the above results, it could be verified that as the amount of water for dissolution increases, the hardness decreases.

According to the present invention, by using a starch adhesive (a semi-solidifying agent) which contains as the main component a sodium carrageenan in which the total amount of potassium, calcium and magnesium ions is 5.0% by weight or less, for the first time, gelatinization is promptly achieved without resort to a heat-dissolution treatment of the starch adhesive, which is one of the major disadvantages of the conventional method, and moreover, by dissolving it in cold water. Further, when it is administered from a syringe, there is no water liberation. Therefore, in the medical field for which urgency is required and in which there is no time to spare, an enteral nutrient can be infused into the body simply and accurately within a short period of time.

Further, according to the present invention, by adding and using a starch adhesive containing as the main component a sodium carrageenan in an enteral nutrient, the formation of a solid matter having such a special property that the enteral nutrient has fluidity before and after the infusion thereof into the body and is not liquefied at the body temperature in a state of being retained in the body was successful for the first time. Further, this special solid matter has an excellent characteristic in which it does not flow back, for example, from the stomach to the esophagus or it does not cause a leak of a nutrient from a fistula, and further it prevents the onset of diarrhea because the administration rate thereof is not fast but moderate.

In this way, because the solid matter according to the present invention is an extremely unique matter having a special property and an excellent characteristic, it is also represented by a "semi-solid matter" in order to clearly distinguish it from a mere gelatinized matter or a solid matter. Therefore, because.a starch adhesive (or a gelatinizing agent, a solidifying agent or a thickening agent: a sodium carrageenan) to be used for producing this semi-solid matter is also a unique matter, this is also represented by a "semi-solidifying agent" in this description in order to' clearly distinguish it from a mere starch adhesive. However, the detailed definitions of these matters are as described above.

Further, according to the present invention, the viscosity of a liquid matter can be easily adjusted by adding the semi-solidifying agent as defined in the present claims to the liquid matter with low to no viscosity such as a liquid food, therefore, it is very useful in the medical or nursing-care field for which rapid operation and easiness of handling are required.

## Claims

1. A semi-solidified enteral nutrient, which is an enteral nutrient to be infused into the body in tube feeding infusion, **characterized by** being formed into a semi-solid matter using a sodium carrageenan, in which the total amount of potassium, calcium and magnesium ions is 5.0% by weight or less, as a semi-solidifying agent such that the enteral nutrient has fluidity before and after the infusion thereof into the body and is not liquefied at the body temperature in a state of being retained in the body.

2. The enteral nutrient according to claim 1, **characterized in that** the semi-solidifying agent is any one or two or more of carrageenans selected from sodium iota-carrageenan, sodium kappa-carrageenan and sodium lambda-carrageenan.

3. The enteral nutrient according to claim 1 or 2, **characterized in that** the semi-solidifying agent is a carrageenan which is hydrated with water at 45°C or below.

4. The enteral nutrient according to any one of claims 1 to 3, **characterized in that** the semi-solidifying agent further contains a protein.

5. The enteral nutrient according to claim 4, **characterized in that** the protein is casein.

6. The enteral nutrient according to any one of claims 1 to 5, **characterized in that** the semi-solidifying agent further contains at least one member selected from lipids, carbohydrates, dietary fibers, vitamins and minerals.

7. The enteral nutrient according to any one of claims 1 to 6, **characterized in that** the semi-solidifying agent further contains at least one member selected from dextrin, starch, xanthan gum, guar gum, locust bean gum and tara gum.

8. The enteral nutrient according to any one of claims 1 to 7, **characterized in that** the tube feeding infusion is at least one method selected from nasogastric feeding, transgastric feeding and enteric feeding.

9. A method of preparing a semi-solidified enteral nutrient according to any one of claims 1 to 8, which is an enteral nutrient to be infused into the body in tube feeding infusion, comprising the step of employing a sodium carrageenan, in which the total amount of potassium, calcium and magnesium ions is 5.0% by weight or less, as a semi-solidifying agent to form a semi-solid matter such that the enteral nutrient has fluidity before and after the infusion thereof into the body and is not liquefied at the body temperature in a state of being retained in the body.

10. Use of a sodium carrageenan, in which the total amount of potassium, calcium and magnesium ions is 5.0% by weight or less, as a semi-solidifying agent for a semi-solidified enteral nutrient according to any one of claims 1 to 8, which is an enteral nutrient to be infused into the body in tube feeding infusion, which is formed into a semi-solid matter using the sodium carrageenan as a semi-solidifying agent such that the enteral nutrient has fluidity before and after the infusion thereof into the body and is not liquefied at the body temperature in a state of being retained in the body.

## Patentansprüche

1. Ein halbverfestigter enteraler Nährstoff, welcher ein enteraler Nährstoff ist, der in den Körper in einer Sondennahrungsinfusion infundiert werden soll, **dadurch gekennzeichnet, dass** dieser durch Verwendung eines Natrium-Carrageens, in welchem die Gesamtmenge an Kalium-, Calcium- und Magnesiumionen 5,0 Gew. % oder weniger beträgt, als ein Halbverfestigungsmittel in die Form eines halbfesten Materials gebracht wird, sodass der enterale Nährstoff vor und nach dessen Infusion in den Körper Fluidität aufweist und bei Körpertemperatur nicht verflüssigt wird, wenn er im Körper vorhanden ist.

2. Der enterale Nährstoff gemäß Anspruch 1, **dadurch gekennzeichnet, dass** das Halbverfestigungsmittel ein oder zwei oder mehr Carrageene ist, welche ausgewählt sind aus Natrium-iota-Carrageen, Natrium-kappa-Carrageen und Natrium-lambda-Carrageen.

3. Der enterale Nährstoff gemäß Anspruch 1 oder 2, **dadurch gekennzeichnet, dass** das Halbverfestigungsmittel ein Carrageen ist, welches mit Wasser bei 45°C oder weniger hydratisiert wird.

4. Der enterale Nährstoff gemäß einem der Ansprüche 1 bis 3, **dadurch gekennzeichnet, dass** das Halbverfestigungsmittel zusätzlich ein Protein enthält.

5. Der enterale Nährstoff gemäß Anspruch 4, **dadurch gekennzeichnet, dass** das Protein Kasein ist.

6. Der enterale Nährstoff gemäß einem der Ansprüche 1 bis 5, **dadurch gekennzeichnet, dass** das Halbverfestigungsmittel zusätzlich mindestens einen Bestandteil ausgewählt aus Lipiden, Kohlenhydraten, Ballaststoffen, Vitaminen und Mineralien enthält.

7. Der enterale Nährstoff gemäß einem der Ansprüche 1 bis 6, **dadurch gekennzeichnet, dass** das Halbverfestigungsmittel zusätzlich mindestens einen Bestandteil ausgewählt aus Dextrin, Stärke, Xanthan, Guargummi, Johannisbrotkernmehl und Tarakernmehl enthält.

8. Der enterale Nährstoff gemäß einem der Ansprüche 1 bis 7, **dadurch gekennzeichnet, dass** die Sondennahrungsinfusion mindestens ein Verfahren ausgewählt aus nasogastraler Ernährung, transgastrischer Ernährung und enteraler Ernährung ist.

9. Ein Verfahren zur Herstellung eines halbverfestigten enteralen Nährstoffs gemäß einem der Ansprüche 1 bis 8, welcher ein enteraler Nährstoff, ist, der in den Körper in einer Sondennahrungsinfusion infundiert werden soll, umfassend den Schritt des Verwendens eines Natrium-Carrageens, in welchem die Gesamtmenge an Kalium-, Calcium- und Magnesiumionen 5,0 Gew. % oder weniger beträgt, als ein Halbverfestigungsmittel zur Bildung eines halbfesten Materials, sodass der enterale Nährstoff vor und nach dessen Infusion in den Körper Fluidität aufweist und bei Körpertemperatur nicht verflüssigt wird, wenn er im Körper vorhanden ist.

10. Verwendung eines Natrium-Carrageens, in welchem die Gesamtmenge an Kalium-, Calcium- und Magnesiumionen 5,0 Gew. % oder weniger beträgt, als ein Halbverfestigungsmittel für einen halbverfestigten enteralen Nährstoff gemäß einem der Ansprüche 1 bis 8, welcher ein enteraler Nährstoff ist, der in den Körper in einer Sondennahrungsinfusion infundiert werden soll, und welcher durch Verwendung des Natrium-Carrageens als ein Halbverfestigungsmittel in die Form eines halbfesten Materials gebracht wird, sodass der enterale Nährstoff vor und nach dessen Infusion in den Körper Fluidität aufweist und bei Körpertemperatur nicht verflüssigt wird, wenn er im Körper vorhanden ist.

## Revendications

1. Nutriment entéral semi-solidifié, qui est un nutriment entéral destiné à être introduit dans le corps par infusion d'alimentation par sonde, **caractérisé en ce qu'**il est converti en matière semi-solide à l'aide d'une carraghénine sodique, dans laquelle la quantité totale d'ions potassium, calcium et magnésium est de 5,0 % en poids ou moins, à titre d'agent semi-solidifiant de façon que le nutriment entéral ait une certaine fluidité avant et après son infusion dans le corps et ne soit pas liquéfié à la température corporelle sous un état susceptible d'être retenu dans le corps.

2. Nutriment entéral selon la revendication 1, **caractérisé en ce que** l'agent semi-solidifiant est une carraghénine quelconque ou deux carraghénines ou plus choisies parmi la iota-carraghénine sodique, la kappa-carraghénine sodique et la lambda-carraghénine sodique.

3. Nutriment entéral selon la revendication 1 ou 2, **caractérisé en ce que** l'agent semi-solidifiant est une carraghénine qui est hydratée avec de l'eau à 45°C ou à une température inférieure.

4. Nutriment entéral selon l'une quelconque des revendications 1 à 3, **caractérisé en ce que** l'agent semi-solidifiant contient en outre une protéine.

5. Nutriment entéral selon la revendication 4, **caractérisé en ce que** la protéine est une caséine.

6. Nutriment entéral selon l'une quelconque des revendications 1 à 5, **caractérisé en ce que** l'agent semi-solidifiant contient en outre un composant choisi parmi les lipides, les glucides, les fibres diététiques, les vitamines et les minéraux.

7. Nutriment entéral selon l'une quelconque des revendications 1 à 6, **caractérisé en ce que** l'agent semi-solidifiant contient en outre au moins un composant choisi parmi la dextrine, l'amidon, la gomme xanthane, la gomme guar, la gomme de caroube et la gomme tara.

8. Nutriment entéral selon l'une quelconque des revendications 1 à 7, **caractérisé en ce que** l'infusion d'alimentation par sonde est au moins une méthode choisie parmi l'alimentation nasogastrique, l'alimentation transgastrique et l'alimentation entérale.

9. Méthode de préparation d'un nutriment entéral semi-solidifié selon l'une quelconque des revendications 1 à 8, qui est un nutriment entéral destiné à être introduit dans le corps par infusion d'alimentation par sonde, comprenant les étapes d'utilisation d'une carraghénine sodique, dans laquelle la quantité totale d'ions potassium, calcium et magnésium est de 5,0 % en poids ou moins, à titre d'agent semi-solidifiant pour former une matière semi-solide de façon que le nutriment entéral ait une certaine fluidité avant et après son infusion dans le corps et ne soit pas liquéfié à la température corporelle sous un état susceptible d'être retenu dans le corps.

10. Utilisation d'une carraghénine sodique, dans laquelle la quantité totale d'ions potassium, calcium et magnésium est de 5,0 % en poids ou moins, à titre d'agent semi-solidifiant pour nutriment entéral selon l'une quelconque des revendications 1 à 8, qui est un nutriment entéral destiné à être introduit dans le corps par une infusion d'alimentation par sonde, qui est converti en matière semi-solide à l'aide de la carraghénine sodique à titre d'agent semi-solidifiant de façon que le nutriment entéral ait une certaine fluidité avant et après son infusion dans le corps et ne soit pas liquéfié à la température corporelle sous un état susceptible d'être retenu dans le corps.
